(19) **Europäisches Patentamt**
**European Patent Office**
**Office européen des brevets**

(11) **EP 0 960 526 B1**

(12) **FASCICULE DE BREVET EUROPEEN**

(45) Date de publication et mention de la délivrance du brevet:
**10.12.2014 Bulletin 2014/50**

(21) Numéro de dépôt: **98908178.1**

(22) Date de dépôt: **12.02.1998**

(51) Int Cl.:
*H04N 5/335* (2011.01)   *G01T 1/24* (2006.01)

(86) Numéro de dépôt international:
**PCT/FR1998/000272**

(87) Numéro de publication internationale:
**WO 1998/036579 (20.08.1998 Gazette 1998/33)**

(54) **DISPOSITIF ET PROCEDE DE TRAITEMENT DE SIGNAUX D'UN DETECTEUR DE RAYONNEMENTS A SEMI-CONDUCTEURS**

VORRICHTUNG UND VERFAHREN ZUR SIGNALVERARBEITUNG EINES HALBLEITERSTRAHLUNGSDETEKTORS

DEVICE AND METHOD FOR PROCESSING SIGNALS OF A RADIATION DETECTOR WITH SEMICONDUCTORS

(84) Etats contractants désignés:
**DE GB IT**

(30) Priorité: **14.02.1997 FR 9701745**

(43) Date de publication de la demande:
**01.12.1999 Bulletin 1999/48**

(73) Titulaire: **Commissariat à l'Énergie Atomique et aux Énergies Alternatives 75015 Paris (FR)**

(72) Inventeurs:
- **MESTAIS, Corinne F-38660 La Terrasse (FR)**

- **CHAPUIS, Alain F-38950 Saint Martin le Vinoux (FR)**
- **MONNET, Olivier F-38210 Tullins (FR)**
- **LEBRUN, François F-94500 Champigny sur Marne (FR)**

(74) Mandataire: **Ahner, Philippe et al BREVALEX 95, rue d'Amsterdam 75378 Paris Cedex 8 (FR)**

(56) Documents cités:
**EP-A- 0 589 467      EP-A- 0 626 590
EP-A- 0 637 759      US-A- 5 440 130**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

Domaine technique

La présente invention se rapporte à un dispositif et à un procédé de traitement de signaux d'un détecteur de rayonnements à semi-conducteurs. L'invention s'applique plus précisément à des détecteurs tels que des détecteurs de rayonnement gamma ($\gamma$) comprenant une pluralité de détecteurs élémentaires juxtaposés selon une surface ou un volume de détection.

Un dispositif ou un procédé de traitement de signaux conforme à l'invention peuvent notamment être utilisés dans le domaine de l'imagerie médicale pour des gamma-caméras à détecteurs du type CdTe ou CdZnTe.

Etat de la technique antérieure

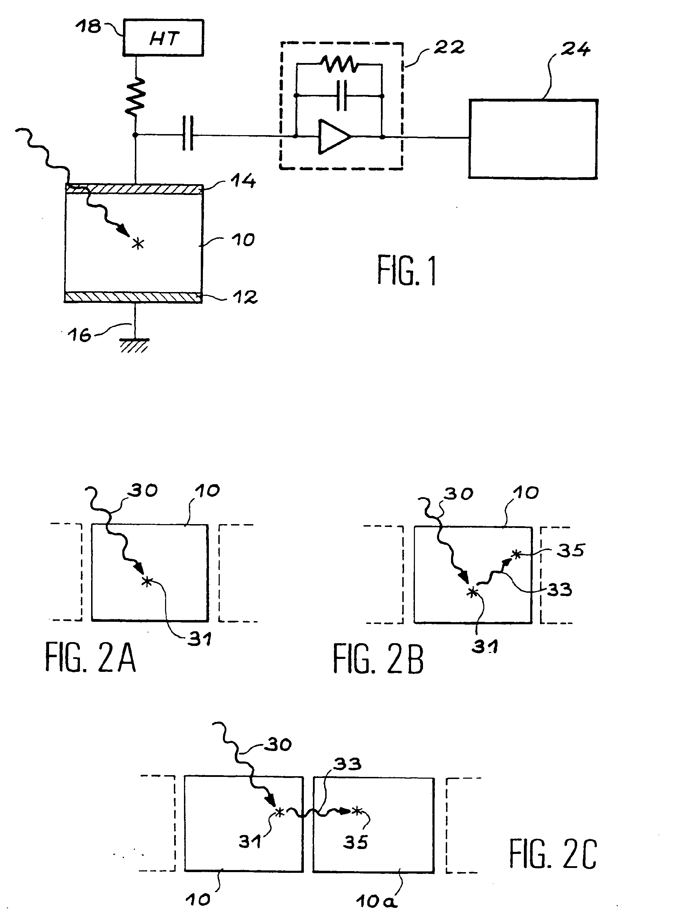

La figure 1 annexée montre le principe de fonctionnement d'un détecteur élémentaire à semi-conducteurs du type CdTe.

Le détecteur en semi-conducteur 10 est équipé de deux électrodes 12, 14 disposées respectivement sur deux faces opposées. Les électrodes 12 et 14 sont à la fois des électrodes de polarisation du détecteur et des électrodes de lecture des signaux de détection.

Pour assurer la polarisation du détecteur les électrodes 12 et 14 sont respectivement reliées à un potentiel de référence de masse 16 et à une source de tension 18. La différence de potentiel appliquée sur les faces opposées du semi-conducteur permettent d'y générer un champ électrique.

Ainsi, lorsqu'un rayonnement atteint le matériau semi-conducteur et y crée des paires électron-trou, ces porteurs électriques ne se recombinent pas mais sont entraînés, sous l'effet du champ électrique, vers les électrodes 12, 14. Sur les électrodes est alors prélevé un signal électrique qui est représentatif de l'énergie cédée par le rayonnement au semi-conducteur.

Comme le montre la figure 1, le signal de détection est prélevé sur l'électrode 14 et, après avoir été mis en forme par un circuit à amplificateur 22, est dirigé vers des moyens de traitement du signal 24.

Dans la suite du texte on désigne par événement toute interaction entre un rayonnement et le détecteur lors de laquelle le rayonnement cède à la matière tout ou partie de son énergie.

Dans une tête de détection comprenant une pluralité de détecteurs élémentaires juxtaposés la localisation géographique des événements est donnée par les coordonnées des détecteurs sur une surface de détection. Ainsi, une image de la source de rayonnement, perçue par la tête de détection peut être établie par des moyens d'imagerie adaptés.

On peut se reporter à ce sujet aux documents (1), (2) et (3) dont les références sont indiquées à la fin de la présente description.

Dans le cas de la détection de rayonnements gamma ($\gamma$) on distingue, à un premier ordre, deux types d'interactions.

On désigne dans la suite du texte par "interaction photoélectrique" le premier type d'interaction lors duquel un rayonnement gamma atteignant le détecteur, appelé "rayonnement incident", cède toute son énergie au matériau du détecteur.

Par ailleurs, on désigne par "interaction compton" un deuxième type d'interaction lors duquel un rayonnement incident ne cède qu'une partie de son énergie.

Dans la suite du texte on désigne par rayonnement incident le rayonnement provenant d'une cible d'observation et atteignant le détecteur. On désigne par ailleurs par "rayonnement induit" le rayonnement résultant d'une interaction compton lors de laquelle le rayonnement incident n'a cédé qu'une partie de son énergie. Le rayonnement induit peut également interagir avec la matière et lui céder son énergie. Dans ce cas, l'énergie totale du rayonnement incident est cédée en plusieurs (généralement deux) interactions.

Lorsqu'un rayonnement électromagnétique tel qu'un rayonnement gamma atteint une tête de détection formée d'une pluralité de détecteurs élémentaires juxtaposés on distingue trois cas différents de détection représentés très schématiquement aux figures 2A à 2C. Pour des raisons de simplification seuls les détecteurs élémentaires sont représentés sur ces figures.

Dans le premier cas, illustré à la figure 2A, l'interaction d'un rayonnement 30 avec le semi-conducteur 10 du détecteur est une interaction de type photoélectrique telle que décrite ci-dessus. Le rayonnement incident 30 cède toute son énergie en une seule interaction repérée avec la référence 31. Le signal de détection obtenu correspond alors à l'énergie totale du rayonnement.

Dans un deuxième cas, illustré à la figure 2B, l'interaction est une interaction compton. Le rayonnement incident 30 cède une partie de son énergie lors d'une première interaction repérée par la référence 31 et un rayonnement induit 33 apparaît. Le rayonnement induit 33 cède à son tour son énergie lors d'une deuxième interaction qui, dans cet exemple est de type photoélectrique repérée avec la référence 35. On note qu'à la fois la première et la deuxième interaction ont lieu dans le même détecteur élémentaire.

Ainsi, comme ces deux interactions sont quasiment simultanées, le signal fourni aux bornes du détecteur correspond finalement à la somme des énergies cédées lors des première et deuxième interactions, c'est-à-dire à l'énergie du rayonnement incident 30.

Dans un troisième cas illustré à la figure 2C, l'énergie du rayonnement incident 30 est également cédée lors de deux interactions 31 et 35. Toutefois à la différence de la figure 2B, les deux interactions 31 et 35 n'ont pas lieu dans le même détecteur mais dans deux détecteurs voisins 10 et 10a de la tête de détection.

Le signal délivré par le premier détecteur 10 dans lequel a lieu la première interaction correspond à l'énergie cédée lors de la première interaction, c'est-à-dire l'énergie du rayonnement incident 31 diminuée de l'énergie du rayonnement induit. Le signal délivré par le deuxième détecteur 10a ne correspond qu'à l'énergie du rayonnement induit, cédée à la matière lors de la deuxième interaction. Finalement aucun des signaux des premier et second détecteurs 10, 10a ne reflète dans ce cas l'énergie du rayonnement incident.

En imagerie médicale, on injecte généralement à un patient un isotope radioactif émettant des rayonnements gamma à une énergie connue et déterminée.

Les rayonnements gamma émis dans le patient peuvent également interagir avec le tissu environnant de l'organe examiné du patient et y provoquer des interactions de type compton telles que décrites.

Ainsi, le rayonnement provenant du patient et atteignant la tête de détection d'une gamma-caméra comporte non seulement le rayonnement de l'isotope avec l'énergie déterminée et connue mais aussi des rayonnements induits, ou rayonnements "compton" dont l'énergie est inférieure.

Pour éviter la prise en compte des rayonnements induits ou compton produits dans le patient, et considérés comme parasites, on effectue à la sortie des détecteurs une discrimination en énergie de façon à ne retenir que les signaux correspondant à l'énergie connue de l'isotope radioactif injecté au patient, c'est-à-dire correspondant à des rayonnements dits "utiles".

On comprend ainsi que le cas d'une détection d'un rayonnement telle que décrite au sujet de la figure 2C est particulièrement gênant.

En effet, lorsqu'un rayonnement incident dont l'énergie est égale à l'énergie déterminée correspondant à l'isotope injecté au patient, produit deux interactions dans deux détecteurs différents, le signal reçu de chaque détecteur correspond à une énergie inférieure à celle du rayonnement incident comme indiqué ci-dessus. Or, ces signaux sont alors éliminés lors de la discrimination en énergie évoquée précédemment.

Ainsi, la contribution d'un rayonnement "utile" est éliminée par erreur comme s'il s'agissait d'un rayonnement provenant d'une interaction de type compton dans le patient.

En raison du fait qu'une dose de produit radioactif injectée au patient doit pour des raisons de santé être relativement limitée, on comprend qu'il convient de ne pas éliminer un trop grand nombre de rayonnements incidents utiles. En effet, comme le temps d'acquisition de données pour former une image médicale ne peut pas être trop longue, notamment pour des raisons de confort du patient, un nombre trop limité de rayonnements utiles conduit à une détérioration de la qualité de l'image finalement obtenue.

La présente invention se propose donc de résoudre le problème de la perte de l'information liée aux rayonnements qui interagissent dans deux détecteurs élémentaires distincts de la tête de détection.

La demande de brevet EP-A-0 589 467 décrit un dispositif d'imagerie médicale capable d'effectuer une correction des informations collectées afin de tenir compte d'une dispersion des interactions, due notamment à l'effet Compton. En particulier, le dispositif comporte un détecteur, tel qu'un scintillateur, associé à des photomultiplicateurs aptes à délivrer des informations de position (en X, Y) et d'énergie des interactions détectées. Une image sous la forme d'une matrice de pixels est formée à partir de ces informations. Pour chaque pixel on prend en compte une contribution pondérée d'un ensemble d'interactions ayant lieu sensiblement simultanément et correspondant à ce pixel. Le poids attribué à chaque interaction dépend de son énergie. Ce dispositif permet de délivrer pour chaque rayonnement incident une donnée représentative de l'ensemble des interactions induites par ce rayonnement.

Exposé de l'invention

La présente invention a pour objet un dispositif de traitement de signaux de détection d'interactions pour un détecteur de rayonnements à semi-conducteurs comprenant une pluralité de détecteurs élémentaires juxtaposés selon une surface ou un volume de détection sensibles aux énergies cédées, en une ou plusieurs interactions, par les rayonnements dits rayonnements incidents, et aptes à délivrer des signaux de détection d'interaction, caractérisé en ce que le dispositif comprend des moyens dits de traitement pour délivrer en réponse à chaque rayonnement incident détecté au moins une donnée d'énergie correspondant à la somme des énergies cédées lors de chaque interaction induite par ledit rayonnement incident sur un détecteur élémentaire principal et des énergies des interactions ayant lieu sensiblement au même moment sur des détecteurs élémentaires voisins au détecteur principal et en ce que les moyens de traitement comportent :

- des premiers moyens pour associer à chaque signal de détection d'interaction d'un détecteur élémentaire des données de position de l'interaction, d'énergie de l'interaction et de date de l'interaction,
- des moyens de sélection pour sélectionner des interactions ayant une même donnée de date d'interaction et des données de position d'interaction correspondant à des détecteurs élémentaires voisins de la surface ou du volume de détection, et
- des moyens sommateurs pour additionner les données d'énergie des interactions sélectionnées et établir une donnée dite de somme d'énergie correspondant à la somme des énergies cédées lors de chaque interaction induite par le rayonnement incident.

On entend par surface de détection aussi bien une surface de détection plane qu'une surface de détection qui n'est pas plane. Par ailleurs, on considère qu'un volume de détection peut comporter plusieurs surfaces de détection éventuellement superposées.

Un rayonnement incident peut interagir soit avec un seul détecteur élémentaire soit avec un détecteur élémentaire et avec les détecteurs élémentaires voisins.

Grâce aux moyens dits de gestion d'information, l'ensemble de l'énergie du rayonnement incident est pris en compte, quel que soit le type d'interaction avec les détecteurs.

Ainsi, tout rayonnement incident ayant l'énergie correspondant à l'isotope injecté au patient peut être pris en compte indépendamment du fait qu'il donne lieu à une seule ou à plusieurs interactions dans des détecteurs élémentaires distincts.

Les données de position sont déterminées en fonction de l'emplacement des détecteurs élémentaires dans la surface ou le volume de détection. A cet effet, les premiers moyens peuvent comporter au moins un circuit apte à délivrer les données de position en fonction d'une localisation dans la surface ou le volume de détection du détecteur élémentaire ayant détecté ladite interaction et fourni le signal.

A titre d'exemple, dans une tête de détection de type matricielle, chaque détecteur peut être repéré par deux coordonnées de position. Ces coordonnées sont alors affectées en tant que données de position à chaque interaction ayant lieu dans ce détecteur.

Les données d'énergie d'interaction correspondent à l'énergie cédée lors de chaque interaction dans un détecteur donné. Lorsque deux interactions provenant d'un même rayonnement incident ont lieu dans un même détecteur élémentaire, la donnée d'énergie correspond automatiquement à la somme des énergies cédées par ces deux interactions, en raison notamment de leur quasi-simultanéité.

Pour déterminer les données d'énergie, en particulier lorsque les détecteurs sont du type CdTe, les premiers moyens peuvent comporter :

- au moins un circuit pour former des données d'amplitude et de temps de montée des signaux de détection, et
- au moins un circuit dit de correction pour calculer une donnée d'énergie d'interaction fonction des données d'amplitude et de temps de montée des signaux de détection.

Pour une description plus détaillée du principe de fonctionnement des circuits de correction, on peut se reporter aux documents (1), (2) et (3) déjà mentionnés.

Enfin, la donnée de date de l'interaction permet d'apprécier la simultanéité des interactions. Pour établir cette donnée, les premiers moyens peuvent comporter au moins un compteur, piloté par une horloge, pour délivrer une donnée de date d'interaction correspondant à chaque signal de détection.

Comme indiqué précédemment, lorsqu'un rayonnement incident provoque deux ou plusieurs interactions, ces interactions sont quasiment simultanées et ont lieu soit dans un même détecteur élémentaire, soit dans des détecteurs plus proches voisins.

Ainsi, lorsque deux interactions ont lieu quasi-simultanément et dans des détecteurs voisins, on considère qu'ils proviennent d'un même rayonnement incident.

Le cas où deux interactions dues à des rayonnements incidents distincts ont lieu simultanément dans des détecteurs élémentaires voisins est très peu probable. Ce cas n'est ainsi pas pris en compte. Dans le domaine d'énergie considéré pour l'imagerie médicale (40-600 keV) et pour des détecteurs élémentaires avec une dimension caractéristique de l'ordre de quelques millimètres, on considère que les détecteurs sont voisins lorsqu'ils ont une frontière commune. On ne prend ainsi en compte que les détecteurs "plus proches voisins".

Dans une réalisation particulière du dispositif de l'invention, les moyens de sélection peuvent comporter une mémoire présentant une pluralité de pages respectivement associées à une pluralité de périodes de temps ou de dates successives, des moyens d'écriture des données d'énergie et de position de chaque interaction respectivement à une page dont la période de temps comprend la date de ladite interaction et des moyens de lecture, page par page, des données écrites dans la mémoire.

A titre d'exemple, la période de temps associée à chaque page peut correspondre à la durée séparant deux impulsions successives d'une horloge, c'est-à-dire deux dates successives.

De plus, les moyens de sélection peuvent comporter des comparateurs de données de position. Ces comparateurs sont alors prévus pour comparer les données de positions inscrites sur une même page de la mémoire, c'est-à-dire qui correspondent à des événements ayant sensiblement la même date d'interaction. Grâce aux comparateurs, il est possible de vérifier si des interactions simultanées proviennent ou non de détecteurs élémentaires voisins.

Le dispositif de l'invention peut comporter en outre des deuxièmes moyens pour délivrer en réponse à chaque rayonnement incident une donnée de position sur la surface ou le volume de détection.

Ces deuxièmes moyens peuvent être équipés d'un calculateur capable de calculer ladite donnée de position du rayonnement incident comme une combinaison linéaire des données de position des interactions quasi-simultanées et ayant lieu dans des détecteurs élémentaires voisins. Le calcul de la donnée de position du rayonnement incident peut être pondéré en fonction des données d'énergie des interactions sélectionnées.

Dans une mise en oeuvre simplifiée, la donnée de position du rayonnement incident peut se résumer à l'une des positions de l'une des interactions, par exemple celle de l'interaction dont l'énergie est la plus importante. La donnée de position du rayonnement incident peut aussi être calculée comme une combinaison de type barycentrique tenant compte de toutes les données de position des interactions induites par ce rayonnement incident.

La présente invention a également pour objet un détecteur de rayonnement comprenant une pluralité de détecteurs élémentaires juxtaposés selon la surface ou le volume de détection et un dispositif de traitement de signaux de détection des détecteurs élémentaires tel que décrit ci-dessus.

Selon des réalisations particulières, la pluralité des détecteurs peut être réalisée à partir d'un ou plusieurs cristaux semi-conducteurs, par exemple du type CdTe ou CdZnTe.

Selon une première possibilité, chaque détecteur élémentaire comporte un matériau de semi-conducteur et des électrodes de polarisation et de lecture ménagées sur des faces opposées du matériau.

Selon une variante, le détecteur peut aussi comporter une pluralité d'électrodes séparées et juxtaposées sur au moins une face du matériau, chaque électrode formant avec une portion du matériau un détecteur élémentaire.

L'invention a également pour objet une gamma-caméra comportant un détecteur de rayonnement et un système de formation d'images tels que décrits ci-dessus.

Enfin, l'invention a pour objet un procédé de traitement de signaux de détection d'interactions d'un détecteur de rayonnements à semi-conducteurs comprenant une pluralité de détecteurs élémentaires juxtaposés selon une surface ou un volume de détection, sensibles aux énergies cédées, en une ou plusieurs interactions, par les rayonnements, dits rayonnements incidents, et aptes à délivrer des signaux de détection d'interaction, caractérisé en ce que :

- on associe à chaque signal de détection d'interaction d'un détecteur élémentaire des données de position de l'interaction, d'énergie de l'interaction et de date de l'interaction,
- on sélectionne des interactions ayant une même donnée de date d'interaction et des données de position d'interaction correspondant à des détecteurs élémentaires voisins de la surface ou du volume de détection, et
- on additionne les données d'énergie des interactions sélectionnées et on établit une donnée dite de somme d'énergie correspondant à la somme des énergies cédées lors de chaque interaction induite par le rayonnement incident.

D'autres caractéristiques et avantages de l'invention ressortiront mieux de la description qui va suivre, en référence aux figures des dessins annexés, donnée à titre purement illustratif et non limitatif.

Brève description des figures

- La figure 1, déjà décrite, est un schéma de principe de fonctionnement d'un détecteur à semi-conducteurs d'une tête de détection.
- Les figures 2A, 2B et 2C, déjà décrites, sont des vues schématiques de détecteurs à semi-conducteurs et illustrent trois types d'interactions d'un rayonnement incident avec le matériau semi-conducteur des détecteurs.

- La figure 3 est une vue schématique d'une tête de détection planaire utilisable avec le dispositif de l'invention pour former une gamma-caméra.
- La figure 4 est une vue schématique d'une autre tête de détection également utilisable avec le dispositif de l'invention.
- La figure 5 est un schéma de fonctionnement général du dispositif de l'invention.
- La figure 6 est un schéma détaillé partiel de moyens de sélection du dispositif de l'invention.

Description détaillée de modes de réalisation de l'invention

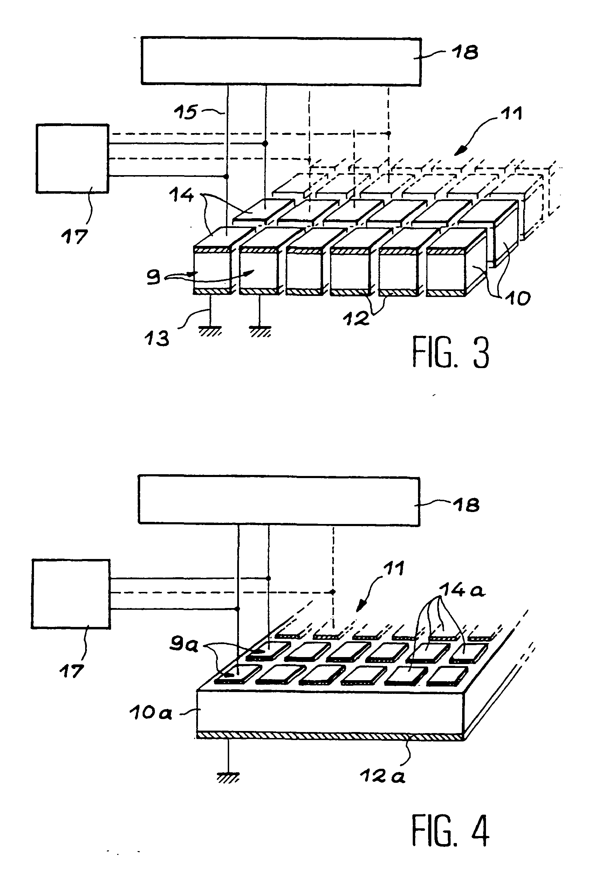

La figure 3 montre un ensemble de détecteurs élémentaires 9 juxtaposés dans un plan de détection 11 pour former une tête de détection.

Chaque détecteur élémentaire est construit à partir d'un bloc de matériau semi-conducteur 10 indépendant. On peut se reporter à ce sujet à la figure 1 déjà décrite.

Une telle tête peut équiper, par exemple, une gamma-caméra comprenant le dispositif de l'invention. Chaque détecteur élémentaire comporte une première électrode 12 disposée sur l'une des faces parallèle au plan de détection et une deuxième électrode 14 disposée sur une face opposée également parallèle au plan de détection. Des conducteurs électriques 13, 15 relient respectivement les électrodes de chaque détecteur élémentaire à un dispositif de traitement des signaux 17 conforme à l'invention représenté de façon non détaillée.

Les conducteurs électriques 13, 15 relient également chaque détecteur élémentaire respectivement à la masse et à une source de polarisation 18.

Selon une variante, illustrée à la figure 4, une pluralité de détecteurs 9a adjacents sont formés dans un seul bloc de matériau de semi-conducteur 10a.

Chaque détecteur 9a est défini par une électrode 14a formée sur une première face du cristal. Ces électrodes 14a sont juxtaposées et séparées les unes des autres de façon à définir un réseau de détecteurs élémentaires 9a.

Une contre-électrode 12a, commune à tous les détecteurs élémentaires 9a est formée sur une face opposée du cristal 10a.

Les électrodes 14a et 12a sont prévues pour polariser les détecteurs 9a à partir d'une source de polarisation 18 et pour collecter les signaux ce détection. Ces signaux sont dirigés vers une unité de traitement 17 conforme à l'invention.

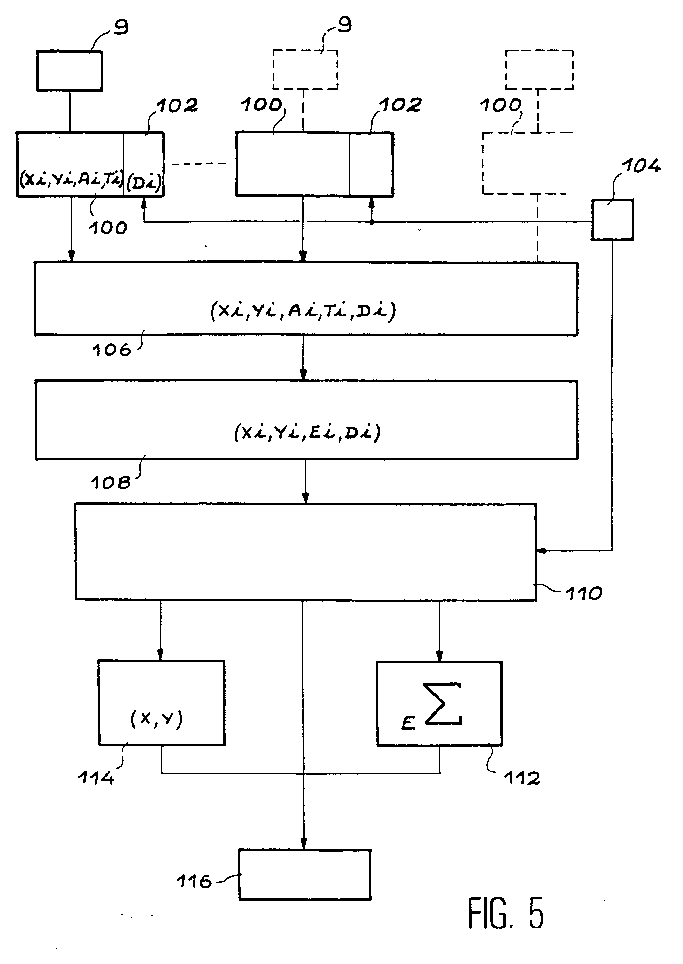

La figure 5 montre de façon plus détaillée les principaux éléments de l'unité de traitement 17.

L'unité de traitement comporte une pluralité de circuits 100 dits de formation de données d'interaction reliés respectivement à chacun des détecteurs élémentaires 9 de la tête de détection.

Les circuits de formation de données ont une double fonction.

Une première fonction est la mise en forme des signaux de détection des détecteurs élémentaires. Une deuxième fonction est d'associer à chaque signal d'intégration un ensemble de données.

Pour chaque signal reçu, les circuits de formation de données 100 délivrent une donnée de position de l'interaction dans le plan de détection, une donnée de temps de montée du signal, une donnée d'amplitude maximum du signal et une donnée de date.

Les données de position sont simplement les coordonnées $(X_i, Y_i)$ selon deux directions qui correspondent à la position sur la surface de détection du $(i^{ème})$ détecteur élémentaire ayant délivré le signal de détection.

Les données de temps de montée et d'amplitude maximum sont directement déterminées à partir du signal de détection mis en forme.

Enfin, la donnée de date est délivrée par un compteur de datation 102 associé à chaque circuit 100.

Les compteurs de datation 102 sont reliés à une horloge de synchronisation 104.

Les compteurs de datation 102 sont, par exemple, des compteurs à 12 bits pilotés par une horloge fonctionnant, par exemple, à une fréquence de 10 MHz.

On définit des cycles de datation. Au cours d'un tel cycle les sorties des compteurs de datation sont augmentés d'une unité à chaque impulsion d'horloge. A la fin de chaque cycle les compteurs sont remis à zéro pour entamer un nouveau cycle de datation.

Dans l'exemple donné, avec des compteurs 102 de 12 bits et une horloge 104 fonctionnant à 10 MHz, la durée d'un cycle de datation est de l'ordre de 410 μséc.

La sortie de chacun des circuits 100 de formation de données d'interaction est reliée à un multiplexeur 106 qui collecte ainsi les données correspondant à tous les événements ayant lieu sur la tête·de·détection. Les données pour un événement i sont notées $(X_i, Y_i, A_i, T_i, D_i)$ où $X_i, Y_i, A_i, T_i$ et $D_i$ désignent respectivement les coordonnées de position, l'amplitude, le temps de montée et la date.

La mesure de l'énergie de l'interaction n'est pas fournie directement par le signal des détecteurs élémentaires. En effet, dans le semi-conducteur, l'énergie déposée par l'interaction provoque la formation de paires électron-trou. Ces porteurs de charge migrent vers les électrodes du détecteur sous l'effet d'un champ électrique maintenu entre ces électrodes. Or, la mobilité des trous étant inférieure à celle des électrons, leur contribution au signal peut être différée selon la profondeur de l'interaction dans la matière du détecteur. Ainsi, le signal n'est pas directement utilisable pour la mesure de l'énergie.

Toutefois, grâce à un circuit 108, dit de correction, l'énergie de l'interaction est calculée à partir des données $A_i$ de l'amplitude du signal et $T_i$ de temps de montée du signal. Le circuit de correction 108 est connecté à la sortie du multiplexeur 106.

Le circuit de correction établit ainsi pour chaque signal une donnée d'énergie notée par la suite Ei pour l'interaction i.

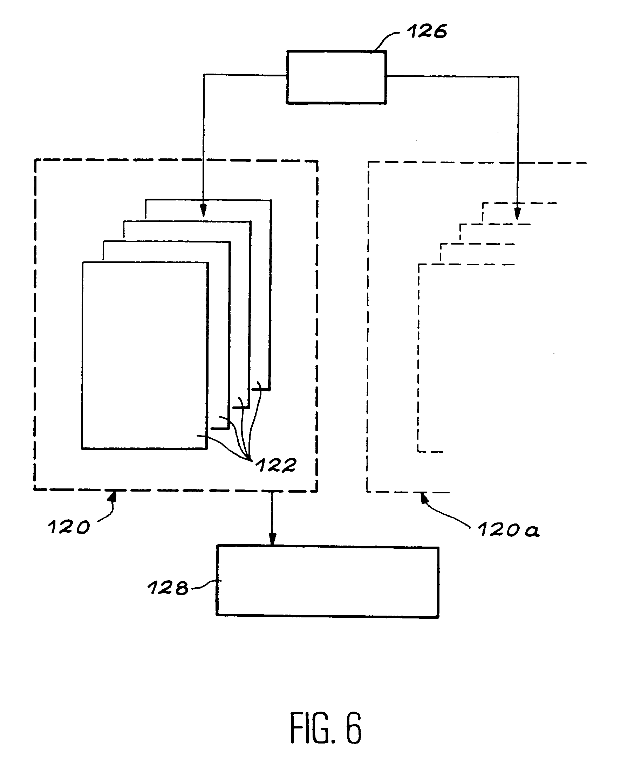

A la sortie du circuit de correction 108, les données $X_i$, $Y_i$, Ei et $D_i$ sont dirigées vers des moyens de sélection 110 dont le fonctionnement est encore expliqué plus loin en référence à la figure 6.

Les moyens de sélection 110 permettent de sélectionner les données des interactions ayant eu lieu sensiblement à la même date et ayant eu lieu dans des détecteurs élémentaires voisins.

Dans une réalisation particulière on ne prend en compte que les détecteurs plus proches voisins. Ceci est le cas notamment lorsque la probabilité est très faible qu'une deuxième interaction ait lieu à une distance supérieure à la taille d'un détecteur élémentaire de la première interaction induite par le même rayonnement.

La sélection des interactions ayant eu lieu dans des détecteurs voisins est effectuée par des circuits à comparateurs.

A titre d'exemple, lorsqu'on considère deux interactions dont les données de position sont $(X_1, Y_1)$ et $(X_2, Y_2)$, on définit les grandeurs $\Delta X$ et $\Delta Y$ suivantes :

$$\Delta X = |X_1 - X_2|$$

$$\Delta Y = |Y_1 - Y_2|$$

où $|X_1-X_2|$ et $|Y_1-Y_2|$ désignent les valeurs absolues des différences.

On estime que les interactions ont lieu dans des détecteurs voisins si les équations suivantes sont vérifiées :

$$\begin{cases} \Delta X = 0 \\ et \\ \Delta Y = 1 \end{cases} \quad ou \quad \begin{cases} \Delta X = 1 \\ et \\ \Delta Y = 0 \end{cases}$$

Il est possible pour une définition différente des détecteurs élémentaires voisins de modifier ces équations, et de prendre par exemple $\Delta X = \Delta Y = 1$ comme critère supplémentaire de définition de détecteurs voisins.

Le calcul des grandeurs $\Delta X$ et $\Delta Y$ et la vérification des équations définissant les détecteurs élémentaires voisins peuvent être effectués par un calculateur ou par un circuit électronique à comparateurs spécialement conçu pour cette tâche.

Les moyens de sélection sont reliés à des moyens sommateurs 112. Ces moyens comportent des circuits additionneurs pour additionner les données d'énergie des interactions sélectionnées, c'est-à-dire des interactions ayant eu lieu quasi-simultanément dans des détecteurs élémentaires voisins.

Ainsi, l'énergie établie pour une interaction multiple de type compton telle que décrite dans la partie introductive du texte est la somme de l'énergie cédée lors de deux ou plusieurs interactions successives et est bien égale à l'énergie totale du rayonnement incident.

Lorsque les données de position ne correspondent pas à des détecteurs voisins, les interactions sont des interactions de type photoélectrique ou Compton seul ayant eu lieu simultanément dans des détecteurs distincts. (On désigne par interaction "Compton seul" une interaction telle que représentée à la figure 2C, dans laquelle la deuxième interaction 35 n'a pas lieu dans un des détecteurs). Ces interactions ne sont pas sélectionnées par les moyens de sélection mais sont traitées classiquement en dirigeant leur données de position et d'énergie vers des moyens d'imagerie 116 connus en soi. Dans ce cas, les données de position et d'énergie de chaque interaction sont également celles du rayonnement ayant provoqué l'interaction.

Les moyens de sélection 110 sont reliés à des moyens 114 pour calculer une donnée de position affectée non pas aux interactions mais au rayonnement incident dont on connaît désormais l'énergie totale.

Les moyens 114 pour calculer la donnée de position comportent un calculateur capable de calculer la donnée de position du rayonnement incident à partir des données de position des interactions auxquelles elle a donnée lieu.

Dans une mise en oeuvre simplifiée, les données de position du rayonnement peuvent être les coordonnées de l'interaction induite dont l'énergie est la plus grande.

Selon un autre exemple, les données de positon X, Y du rayonnement peuvent être calculées selon les formules suivantes :

$$X = \frac{X_1 + X_2}{2} \quad \text{et} \quad Y = \frac{Y_1 + Y_2}{2}$$

Selon une mise en oeuvre plus perfectionnée, le calcul barycentrique peut aussi être pondéré en fonction de l'énergie des interactions. On a alors par exemple :

$$X = \frac{X_1 . E_1 + X_2 . E_2}{E_1 + E_2} \quad \text{et} \quad Y = \frac{Y_1 . E_1 + Y_2 . E_2}{E_1 + E_2}$$

Dans les équations $X_1 Y_1$, $E_1$, $X_2$, $Y_2$, $E_2$ désignent les données de position et d'énergie respectivement d'une première et d'une deuxième interaction provoquées par un rayonnement incident, quasiment à la même date dans des détecteurs voisins.

Les moyens sommateurs 112 et les moyens 114 pour calculer les données de position pour les interactions peuvent être reliés à un dispositif d'imagerie 116.

Le dispositif d'imagerie d'un type connu en soi permet de former une image gamma avec les données de position et éventuellement d'énergie des rayonnements incidents.

La figure 6 illustre schématiquement le fonctionnement d'une partie des moyens de sélection 110. Ces moyens comportent une mémoire vive RAM (Random Access Memory) 120 dans laquelle peuvent être écrites et depuis laquelle peuvent être lues notamment les données de position et d'énergie des interactions détectées.

La mémoire est subdivisée en une pluralité de pages 122. Plus précisément, dans une mise en oeuvre particulière de l'invention, le nombre de pages 122 est égal au nombre de dates différentes possibles dans un cycle de datation. A titre d'exemple, la mémoire 120 peut comporter 4096 pages et chaque page correspond à une date.

Lorsque l'horloge de pilotage 104 fonctionne à une fréquence de 10 MHz, deux dates successives sont séparées dans le temps de 100 nsec.

Lors d'un cycle de datation, les données associées à tous les signaux de détection sont inscrits dans la mémoire 120. L'adresse d'écriture des données est dictée par la donnée de date $D_i$ associée à chaque signal de détection par un compteur de datation 102 (voir figure 5).

Ainsi, toutes les données de toutes les interactions ayant eu lieu à une même date $D_i$ sont écrites dans la mémoire à une même page correspondant à cette date.

Sur la figure 6, la référence 126 indique un registre d'écriture. Ce registre reçoit les données ($X_i$, $Y_i$, $E_i$, $D_i$) des moyens de correction 108 et écrit les données ($X_i$, $Y_i$, $E_i$) à la page $D_i$ dans la mémoire.

Lorsque le cycle de datation est achevé, toutes les dates correspondant aux pages 122 ont défilé sur les compteurs 102 (voir figure 5). Les compteurs sont alors remis à zéro.

La mémoire 120 est ensuite lue page par page par un registre de lecture non représenté. Toutes les données lues à une même page sont considérées comme correspondant à des événements simultanés. Si le nombre d'événements d'une page est supérieur à 1, les données sont dirigées vers des circuits à comparateurs 128 des moyens de sélection, pour vérifier si les données correspondent à des signaux provenant de détecteurs voisins. Lorsque le nombre d'événements dont les données sont à une même page de la mémoire est égal à 1, il s'agit d'une unique interaction de type photoélectrique ou de type Compton seul. Les données sont dirigées directement vers le dispositif d'imagerie 116 (voir figure 5).

Avantageusement, les moyens de sélection peuvent disposer de plusieurs mémoires 120, 120a. Ainsi les mémoires peuvent fonctionner en alternance de sorte que l'une des mémoires peut être adressée pour l'écriture de nouvelles données pendant que des données sont lues dans la seconde mémoire, et réciproquement.

Dans une réalisation pratique du dispositif de traitement de l'invention, l'ensemble des circuits électroniques assurant les fonctions décrites ci-dessus peut être réalisé sous la forme d'un circuit intégré à application spécifique du type ASIC.

DOCUMENTS CITES

*(1)* The 1996 US Workshop on the Physics and Chemistry of II-VI Materials - Las Vegas 1996 "New Developments of CdTe and CdZnTe detectors for X and $\gamma$-ray application" de L. Verger et coll.

**EP 0 960 526 B1**

*(2)* 9th international workshop on room temperature semiconductor X and detectors associated electronics & application "A novel ASIC for readout electronics in semiconductor γ-ray detection" de J.P. Bonnefoy et coll.

*(3)* Nuclear Instruments and Methods in Physics Research A 000 (1996) "A CdTe gamma-camera for the space observatory INTEGRAL : software charge-loss corrections" de F. Lebrun et coll.

## Revendications

**1.** Dispositif de traitement de signaux de détection d'interactions pour un détecteur de rayonnements à semi-conducteurs comprenant une pluralité de détecteurs élémentaires (9, 9a) juxtaposés selon une surface ou un volume de détection (11) sensibles aux énergies cédées, en une ou plusieurs interactions, par les rayonnements dits rayonnements incidents, et aptes à délivrer des signaux de détection d'interaction, **caractérisé en ce que** le dispositif comprend des moyens dits de traitement pour délivrer en réponse à chaque rayonnement incident détecté au moins une donnée d'énergie correspondant à la somme des énergies cédées lors de chaque interaction induite par ledit rayonnement incident sur un détecteur élémentaire principal et des énergies des interactions ayant lieu sensiblement au même moment sur des détecteurs élémentaires voisins au détecteur principal et **en ce que** les moyens de traitement comportent :

- des premiers moyens (100) pour associer à chaque signal de détection d'interaction d'un détecteur élémentaire (9, 9a) des données de position (X, Y) de l'interaction, d'énergie de l'interaction et de date (D) de l'interaction,
- des moyens de sélection (110) pour sélectionner des interactions ayant une même donnée de date d'interaction et des données de position (X, Y) d'interaction correspondant à des détecteurs élémentaires voisins de la surface ou du volume de détection, et
- des moyens sommateurs (112) pour additionner les données d'énergie des interactions sélectionnées et établir une donnée dite de somme d'énergie correspondant à la somme des énergies cédées lors de chaque interaction induite par le rayonnement incident.

**2.** Dispositif selon la revendication 1, **caractérisé en ce qu'**il comporte en outre des deuxièmes moyens (114) pour délivrer en réponse à chaque rayonnement incident une donnée de position sur la surface ou dans le volume de détection.

**3.** Dispositif selon la revendication 2, dans lequel les moyens pour délivrer en réponse à chaque rayonnement incident une donnée de position, comportent un calculateur capable de calculer ladite donnée de position du rayonnement incident comme une combinaison linéaire des données de position des interactions fonction des données d'énergie des interactions sélectionnées.

**4.** Dispositif selon la revendication 1, dans lequel les moyens de sélection (110) comportent au moins une mémoire (120, 120a) présentant une pluralité de pages (122) respectivement associées à une pluralité de périodes de temps successives, des moyens d'écriture des données d'énergie et de position de chaque interaction respectivement à une page dont la période de temps comprend la date de ladite interaction et des moyens de lecture, page par page, des données écrites dans la mémoire.

**5.** Dispositif selon la revendication 1, dans lequel les moyens de sélection (110) comportent des comparateurs de données de positions.

**6.** Dispositif selon la revendication 1, dans lequel les premiers moyens comportent au moins un circuit apte à délivrer les données de position en fonction d'une localisation sur la surface ou le volume de détection du détecteur élémentaire ayant détecté ladite interaction.

**7.** Dispositif selon la revendication 1, dans lequel les premiers moyens comportent :

- au moins un circuit (100) pour former des données d'amplitude et de temps de montée des signaux de détection,
- au moins un circuit (108) dit de correction pour calculer une donnée d'énergie d'interaction fonction des données d'amplitude et de temps de montée des signaux de détection.

**8.** Dispositif selon la revendication 1 dans lequel les premiers moyens comportent au moins un compteur (102), piloté par une horloge (104) pour délivrer une donnée de date d'interaction correspondant à chaque signal de détection.

9. Dispositif selon la revendication 6 comprenant une pluralité de circuits (100) pour former des données d'amplitude et de temps de montée des signaux, un tel circuit étant associé à chaque détecteur élémentaire.

10. Dispositif selon la revendication 7, dans lequel un unique circuit de correction (108), commun, est associé à l'ensemble des détecteurs élémentaires (9, 9a) et dans lequel un multiplexeur (106) est connecté entre la pluralité des circuits de formation de données d'amplitude et de temps de montée des signaux et le circuit de correction (108).

11. Détecteur de rayonnement comprenant une pluralité de détecteurs élémentaires (9, 9a) juxtaposés selon une surface ou un volume de détection (11) et un dispositif (17) de traitement de signaux de détection des détecteurs élémentaires conforme à la revendication 1.

12. Détecteur de rayonnement selon la revendication 11, dans lequel chaque détecteur élémentaire comporte un matériau (10) de semi-conducteur de détection et des électrodes (12, 14) de polarisation et de lecture ménagées sur des faces opposées du détecteur (10).

13. Détecteur de rayonnement selon la revendication 11 comportant au moins un matériau semi-conducteur de détection et une pluralité d'électrodes séparées (14a) et juxtaposées sur au moins une face du matériau semiconducteur, chaque électrode formant avec une portion du matériau semiconducteur un détecteur élémentaire (9a).

14. Détecteur selon l'une des revendications 12 ou 13, dans lequel le matériau semi-conducteur est un cristal de CdTe ou CdZnTe.

15. Gamma-caméra comportant un détecteur de rayonnements et un système de formation d'images (116), **caractérisée en ce que** le détecteur de rayonnement est conforme à la revendication 11.

16. Procédé de traitement de signaux de détection d'interactions d'un détecteur de rayonnements à semi-conducteurs comprenant une pluralité de détecteurs élémentaires juxtaposés selon une surface ou un volume de détection, sensibles aux énergies cédées, en une ou plusieurs interactions, par les rayonnements, dits rayonnements incidents, et aptes à délivrer des signaux de détection d'interaction, **caractérisé en ce que** :

- on associe à chaque signal de détection d'interaction d'un détecteur élémentaire (9, 9a) des données de position (X, Y) de l'interaction, d'énergie de l'interaction et de date (D) de l'interaction,
- on sélectionne des interactions ayant une même donnée de date d'interaction et des données de position (X, Y) d'interaction correspondant à des détecteurs élémentaires voisins de la surface ou du volume de détection, et
- on additionne les données d'énergie des interactions sélectionnées et on établit une donnée dite de somme d'énergie correspondant à la somme des énergies cédées lors de chaque interaction induite par le rayonnement incident.

17. Procédé selon la revendication 16, dans le cas d'une surface de détection, dans lequel on établit en outre une donnée de position en réponse à chaque rayonnement incident, la donnée de position correspondant à une combinaison linéaire de données de position des interactions sur la surface de détection en fonction des énergies des interactions induites par le rayonnement.

**Patentansprüche**

1. Vorrichtung zur Verarbeitung von Signalen zur Erfassung von Interaktionen für einen Halbleiter-Strahlungsdetektor, aufweisend eine Mehrzahl von Detektorelementen (9, 9a), die entlang einer Erfassungsfläche oder einem Erfassungsvolumen (11) nebeneinander angeordnet sind und die empfindlich sind für die Energien, welche bei einer oder mehreren Interaktionen durch die Strahlung, die als einfallende Strahlungen bezeichnet werden, abgegeben werden, und geeignet sind, Interaktionserfassungssignale auszugeben, **dadurch gekennzeichnet, dass** die Vorrichtung Einrichtungen aufweist, die als Verarbeitungseinrichtungen bezeichnet werden, die zum Ausgeben, ansprechend auf eine jeweilige erfasste einfallende Strahlung, mindestens einer Energieangabe dienen, welche der Summe der Energien, die während einer jeweiligen Interaktion abgegeben werden, welche durch die auf ein Haupterfassungselement einfallende Strahlung induziert wird, und der Energien der Interaktionen entspricht, die im Wesentlichen gleichzeitig auf den zum Hauptdetektor benachbarten Detektorelementen stattfinden, und dadurch, dass die Verarbeitungseinrichtungen aufweisen:

erste Einrichtungen (100), um mit jedem Interaktionserfassungssignal eines Detektorelements (9, 9a) Angaben einer Position (X, Y) der Interaktion, einer Energie der Interaktion und eines Zeitpunkts (D) der Interaktion zu assoziieren,

Auswähleinrichtungen (110) zum Auswählen von Interaktionen, die eine gleiche Interaktionszeitangabe aufweisen und deren Interaktionspositionsangaben (X, Y) Detektorelementen entsprechen, die zur Erfassungsfläche oder dem Erfassungsvolumen benachbart angeordnet sind, und

Summierungseinrichtungen (112), um die Energieangaben der ausgewählten Interaktionen zu addieren und eine Angabe zu erstellen, die als Energiesumme bezeichnet wird, welche der Summe der Energien entspricht, die während einer jeweiligen durch die einfallende Strahlung induzierten Interaktion abgegeben werden.

2. Vorrichtung nach Anspruch 1, **dadurch gekennzeichnet, dass** sie weiter zweite Einrichtungen (114) aufweist, um, ansprechend auf jede einfallende Strahlung, eine Angabe einer Position auf der Erfassungsfläche oder in dem Erfassungsvolumen auszugeben.

3. Vorrichtung nach Anspruch 2, bei der die Einrichtungen, die zum Ausgeben, ansprechend auf jede einfallende Strahlung, einer Positionsangabe dienen, eine Berechnungseinrichtung aufweisen, die fähig ist, die Angabe einer Position der einfallenden Strahlung zu berechnen, und zwar in der Art einer linearen Kombination von Positionsangaben der Interaktionen, abhängig von Angaben einer Energie der ausgewählten Interaktionen.

4. Vorrichtung nach Anspruch 1, bei der die Auswähleinrichtungen (110) aufweisen: mindestens eine Speichereinrichtung (120, 120a), die eine Mehrzahl von Seiten (122) aufweist, welche jeweils mit einer Mehrzahl von aufeinanderfolgenden Zeiträumen assoziiert sind, Einrichtungen, um Angaben einer Energie und einer Position einer jeweiligen Interaktion jeweils auf eine Seite zu schreiben, deren Zeitraum den Zeitpunkt dieser Interaktion beinhaltet, und Einrichtungen, um Angaben, die in die Speichereinrichtung geschrieben sind, seitenweise zu lesen.

5. Vorrichtung nach Anspruch 1, bei der die Auswähleinrichtungen (110) Vergleichseinrichtungen von Positionsangaben aufweisen.

6. Vorrichtung nach Anspruch 1, bei der die ersten Einrichtungen mindestens eine Schaltung aufweisen, die fähig ist, die Positionsangaben auszugeben, abhängig von einer Lokalisierung auf der Erfassungsfläche oder dem Erfassungsvolumen des Detektorelementes, von dem diese Interaktion erfasst wurde.

7. Vorrichtung nach Anspruch 1, bei der die ersten Einrichtungen aufweisen:

mindestens eine Schaltung (100), um Angaben einer Amplitude und einer Anstiegszeit von Erfassungssignalen zu erzeugen,

mindestens eine Schaltung (108), die als Korrekturschaltung bezeichnet wird, um eine Angabe einer Interaktionsenergie zu berechnen, abhängig von Angaben einer Amplitude und einer Anstiegszeit von Erfassungssignalen.

8. Vorrichtung nach Anspruch 1, bei der die ersten Einrichtungen mindestens eine Zähleinrichtung (102) aufweisen, die durch einen Taktgeber (104) angesteuert wird, um eine Angabe eines einem jeweiligen Erfassungssignal entsprechenden Interaktionszeitpunkts auszugeben.

9. Vorrichtung nach Anspruch 6, die eine Mehrzahl von Schaltungen (100) aufweist, um Angaben einer Amplitude und einer Anstiegszeit von Signalen zu erzeugen, wobei eine derartige Schaltung mit jedem Erfassungselement assoziiert ist.

10. Vorrichtung nach Anspruch 7, bei der eine einzige, gemeinsame, Korrekturschaltung (108) mit der Gesamtheit der Detektorelemente (9, 9a) assoziiert ist und bei der ein Multiplexer (106) zwischen der Mehrzahl von Schaltungen zur Erzeugung von Angaben einer Amplitude und einer Anstiegszeit von Signalen und der Korrekturschaltung (108) angeschlossen ist.

11. Strahlungsdetektor, aufweisend eine Mehrzahl von Detektorelementen (9, 9a), die entlang einer Erfassungsfläche oder einem Erfassungsvolumen (11) nebeneinander angeordnet sind, und eine Vorrichtung (17) zur Verarbeitung von Erfassungssignalen von Detektorelementen gemäß Anspruch 1.

12. Strahlungsdetektor nach Anspruch 11, bei dem jedes Detektorelement ein Halbleiterdetektormaterial (10) sowie Polarisations- und Lese-Elektroden (12, 14) aufweist, die auf entgegengesetzten Flächen des Detektors (10) ausgebildet sind.

13. Strahlungsdetektor nach Anspruch 11, aufweisend mindestens ein Halbleiterdetektormaterial und eine Mehrzahl von separaten Elektroden (14a), die nebeneinander auf mindestens einer Seite des Halbleitermaterials angeordnet sind, wobei jede Elektrode mit einem Abschnitt des Halbleitermaterials ein Detektorelement (9a) bildet.

14. Detektor nach einem der Ansprüche 12 oder 13, bei dem das Halbleitermaterial ein Kristall aus CdTe oder CdZnTe ist.

15. Gammakamera, die einen Strahlungsdetektor und ein Bilderzeugungssystem (116) aufweist, **dadurch gekennzeichnet, dass** der Strahlungsdetektor gemäß Anspruch 11 ausgebildet ist.

16. Verfahren zur Verarbeitung von Signalen zur Erfassung von Interaktionen eines Halbleiter-Strahlungsdetektors, aufweisend eine Mehrzahl von Detektorelementen, die entlang einer Erfassungsfläche oder einem Erfassungsvolumen nebeneinander angeordnet sind und die empfindlich sind für die Energien, welche bei einer oder mehreren Interaktionen durch die Strahlung, die als einfallende Strahlungen bezeichnet werden, abgegeben werden, und geeignet sind, Interaktionserfassungssignale auszugeben, **dadurch gekennzeichnet, dass**
mit jedem Interaktionserfassungssignal eines Detektorelements (9, 9a) Angaben einer Position (X, Y) der Interaktion, einer Energie der Interaktion und eines Zeitpunkts (D) der Interaktion assoziiert werden,
Interaktionen ausgewählt werden, die eine gleiche Interaktionszeitangabe aufweisen und deren Interaktionspositionsangaben (X, Y) Detektorelementen entsprechen, die zur Erfassungsfläche oder dem Erfassungsvolumen benachbart angeordnet sind, und
die Energieangaben der ausgewählten Interaktionen addiert werden und eine Angabe erstellt wird, die als Energiesumme bezeichnet wird, welche der Summe der Energien entspricht, die während einer jeweiligen durch die einfallende Strahlung induzierten Interaktion abgegeben werden.

17. Verfahren nach Anspruch 16, im Fall einer Erfassungsfläche, bei der weiter eine Positionsangabe, ansprechend jeweils auf eine einfallende Strahlung, erstellt wird, wobei die Positionsangabe einer linearen Kombination von Positionsangaben der Interaktionen auf der Erfassungsfläche entspricht, abhängig von den durch die Strahlung induzierten Interaktionsenergien.

**Claims**

1. A device for processing interaction detection signals for a radiation detector with semiconductors that comprises a plurality of elementary detectors (9, 9a) placed side by side along a surface or volume of detection (11) sensitive to the energies given up in one or more interactions by rays called incident rays and which are capable of supplying interaction detection signals, **characterized in that** the device comprises means called processing means to supply, in response to each incident ray detected, at least one energy datum corresponding to the sum of the energies given up during each interaction induced by said incident ray on a main elementary detector and interaction energies having taken place substantially at the same moment on elementary detectors that are neighbors to the main detector and **in that** the processing means comprise :

   - first means (100) to associate with each interaction detection signal of an elementary detector (9, 9a), interaction position data (X, Y), interaction energy data and an interaction date datum (D),
   - selection means (110) to select the interactions that have substantially one and the same interaction date datum and interaction position data (X, Y) that correspond to neighboring elementary detectors in the surface or the volume of detection, and
   - summation means (112) to add the energy data of the selected interactions and to establish a datum
   - called the energy sum datum which corresponds to the sum of the energies given up during each interaction induced by the incident ray.

2. A device according to Claim 1, **characterized in that**, in addition, it comprises second means (114) to supply, in response to each incident ray, a datum of position on the surface or in the volume of detection.

3. A device according to Claim 2, in which, the means of supplying, in response to each incident ray, a position datum, comprise a calculating device capable of calculating said position datum of the incident ray as a linear combination of the interaction position data in relation to the selected interactions energy data.

**4.** A device according to Claim 1, in which, the selection means (110) comprise at least one memory (120, 120a) having a plurality of pages (122) respectively associated with a plurality of successive periods of time, means of writing energy and position data for each interaction respectively on a page whose period of time includes the date of said interaction and means of reading, page by page, the data written into the memory.

**5.** A device according to Claim 1, in which, the selection means (110) comprise position data comparators.

**6.** A device according to Claim 1, in which, the first means comprise at least one circuit capable of supplying position data as a function of a location on the surface or the volume of detection of the elementary detector that has detected said interaction.

**7.** A device according to Claim 1, in which, the first means comprise :

- at least one circuit (100) to form the amplitude and the rise time data for the detection signals
- at least one circuit (108) called a correction circuit to calculate an interaction energy datum as a function of the amplitude and rise time data of the detection signals.

**8.** A device according to Claim 1, in which, the first means comprise at least one counter (102), controlled by a clock (104) to supply an interaction date datum that corresponds to each detection signal.

**9.** A device according to Claim 6 comprising a plurality of circuits (100) to form amplitude and rise time data for the signals, such a circuit being associated with each elementary detector.

**10.** A device according to Claim 7, in which a single common correction circuit (108) is associated with the assembly of elementary detectors (9, 9a) and into which a multiplexer (106) is connected between the plurality of circuits for the formation of the amplitude and rise time data for the signals and the correction circuit (108).

**11.** A radiation detector comprising a plurality of elementary detectors (9, 9a) placed side by side along a surface or a volume of detection (11) and a device (17) for processing detection signals from elementary detectors conforming to Claim 1.

**12.** A radiation detector according to Claim 11, in which each elementary detector includes a semiconductor detection material (10) and polarization and reading
electrodes (12, 14), created on opposite faces of the detector (10).

**13.** A radiation detector according to Claim 11 comprising at least one semiconductor detection material and a plurality of electrodes (14a) separated and placed side by side on at least one face of the semiconductor material, each electrode forming with a portion of the semiconductor material, an elementary detector (9a).

**14.** A detector according to one of Claims 12 or 13, in which the semiconductor material is a crystal of CdTe or CdZnTe.

**15.** A gamma camera comprising a radiation detector and a system for forming images (116) **characterized in that** the radiation detector conforms to Claim 11.

**16.** A method of processing interaction detection signals from a radiation detector with semiconductors comprising a plurality of elementary detectors placed side by side along a surface or a volume of detection, sensitive to the energies given up in one or more interactions by the rays, called incident rays, and capable of supplying interaction detection signals, **characterized in that** :

- one associates with each interaction detection signal from an elementary detector (9, 9a), position data (X, Y) for the interaction, energy for the interaction and a date datum (D) for the interaction
- one selects interactions having one and the same interaction date datum and interaction position data (X, Y) that correspond to elementary detectors that
- are neighbors on the surface or volume of detection, and
- one adds the energy data of the selected interactions and one establishes a datum called an energy sum datum that corresponds to the sum of the energies given up during each interaction induced by the incident ray.

**17.** A method according to Claim 16, in the case of a detection surface, in which, in addition, one establishes a position datum in response to each incident ray, the position datum corresponding to a linear combination of position data for the interactions on the detection surface as a function of energies of the interactions induced by the ray.

FIG. 1

FIG. 2A

FIG. 2B

FIG. 2C

FIG. 3

FIG. 4

FIG. 5

FIG. 6

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0589467 A

**Littérature non-brevet citée dans la description**

- **L. VERGER.** New Developments of CdTe and CdZnTe detectors for X and $\gamma$-ray application. *The 1996 US Workshop on the Physics and Chemistry of II-VI Materials - Las Vegas,* 1996
- **J.P. BONNEFOY.** A novel ASIC for readout electronics in semiconductor $\gamma$-ray detection. *9th international workshop on room temperature semiconductor X and detectors associated electronics & application*
- **F. LEBRUN.** A CdTe gamma-camera for the space observatory INTEGRAL : software charge-loss corrections. *Nuclear Instruments and Methods in Physics Research A 000,* 1996